# EUROPÄISCHE PATENTSCHRIFT

(11) **EP 2 885 014 B1**
(45) Veröffentlichungstag und Bekanntmachung des Hinweises auf die Patenterteilung: **30.06.2021**
(21) Anmeldenummer: 13801975.7
(22) Anmeldetag: 20.08.2013
(51) Int. Cl.: A61L 27/04, A61L 27/32, A61L 27/34, A61L 27/50, A61L 27/54, A61L 27/58

(54) **IMPLANTAT UND VERFAHREN ZU SEINER HERSTELLUNG**
IMPLANT AND METHOD FOR THE PRODUCTION THEREOF
IMPLANT ET PROCÉDÉ DE FABRICATION CORRESPONDANT

(30) Priorität: 20.08.2012 DE 102012016335; 15.03.2013 DE 102013004420
(43) Veröffentlichungstag der Anmeldung: 24.06.2015
(73) Patentinhaber: Kopp, Alexander, 52062 Aachen (DE)
(72) Erfinder: SMEETS, Ralf, 97892 Kreuzwertheim (DE)
(74) Vertreter: Bungartz, Florian
(86) Internationale Anmeldenummer: PCT/DE2013/000466
(87) Internationale Veröffentlichungsnummer: WO 2014/029379

(56) Entgegenhaltungen:
- WO-A1-2008/103101
- DE-A1-102010 008 357
- US-A1- 2006 089 715
- US-A1- 2009 187 258
- US-A1- 2011 301 696
- None

## Beschreibung

Die Erfindung betrifft ein Implantat für die Geweberegeneration und/oder Knochenregeneration sowie ein Verfahren zu seiner Herstellung.

In der Implantologie ist es in vielen Bereichen notwendig fehlenden Knochen neu aufzubauen. Beispielsweise ist dies der Fall in der Oral- und MKG-Chirurgie: Wenn zu wenig Knochen bei einem Patienten vorhanden ist um ein Kieferimplantat mechanisch stabil zu inserieren, wird der Knochen "augmentiert", indem eine künstliche Kavität geschaffen wird und diese mit autologen Knochen oder Knochenersatzmaterialien aufgefüllt wird. Diese werden in der nachfolgenden Aufbauphase in Knochen umgebaut. Wenn dieser Prozess abgeschlossen ist, kann das Implantat sicher gesetzt werden.

Problematisch ist hierbei, dass Epithel- und Bindegewebe schneller wächst als Knochengewebe und Zellen des Paradontalligaments. Deshalb wird versucht zu verhindern, dass die Defekte vorzeitig mit Epithel- und Bindegewebe durchwachsen werden, sodass das Auffüllen bzw. Zuwachsen der Defekte durch Knochen gestört oder verhindert wird. Deshalb werden die mit Knochenersatzmaterial und/oder autologem Knochen gefüllten oder ungefüllten Hohlräume mit einer i.d.R. kollagenbasierten Membran abgedeckt. Die Membran bietet die typische "Zeltdachfunktion" und hält den Hohlraum aufrecht und frei, damit das Zielgewebe wie beispielsweise Knochen einwachsen kann, ohne dass störendes Bindegewebe einwächst. Über der Membran wird die Gingiva, das heißt das Zahnfleisch, wieder verschlossen.

Nach der Regeneration des Defekts (ca. nach 6 Monaten) muss die Membran wieder entnommen werden. Um diesen Eingriff zu vermeiden, wurden resorbierbare Membranen entwickelt. Um diese wiederum zu verbessern, wird vorgeschlagen, die Membranen durch Stützstrukturen zu stabilisieren. Denn die Kollagenmembranen werden sehr weich und instabil, wenn sie nass werden, und sind hierdurch nur schwierig zu platzieren. Außerdem besteht die Gefahr, dass die Membran in den Defekt "fällt", was als Membrankollaps bezeichnet wird. Gleichzeitig will der Operateur die Membran gut modellieren können, um den aufzubauenden Hohlraum bestimmen zu können. Des Weiteren kann durch die Stützfunktion ein "Verrutschen" (Dislokation) der platzierten Membran, insbesondere beim "Verschluss" des Weichgewebes, vermieden werden. Stand der Technik ist z.B. eine Stabilisierung der Membranen mit Titan-Streben wie in der WO 97/32616 beschrieben. Problematisch ist hierbei jedoch, dass dort ein dauerstabiles Material, nämlich Titan, zusammen mit einer eigentlich resorbierbaren Membran eingebracht wird und eine endgültige Entnahme dieser Stützstrukturen aus Titan, trotz Resorption der eigentlichen Membran, wiederum erfolgen muss. US 2006/089715 A1 beschreibt ein expandierbares Implantat, umfassend einen Stützkörper , der aus eine Mehrzahl von verformbaren Elementen besteht die spiralförmig miteinander verbunden sind. Diese Elemente sind aus einem Metall, wie z.B. einer Magnesiumlegierung. Eine Beschichtung umgebt die Elemente.

Aus anders liegenden Anwendungsgebieten sind Implantate beispielsweise vorgeschlagen worden in der US 2011/301696 A1, der DE 10 2011 107 577 A1, der WO 2008/064672 A2, der DE 10 2004 036 954 A1, der WO 2006/077154 A2, der EP 2 578 245 A2 oder dem Artikel Klocke et al.: "EDM machining capabilities of magnesium (Mg) alloy WE43 for medical applications" in SciVerse ScienceDirect, Procedia Engineering 19 (2011) 190-195, abrufbar unter www.sciencedirect.com.

Aufgabe der vorliegenden Erfindung ist es, den Stand der Technik zu verbessern oder ihm eine Alternative zur Seite zu stellen.

Diese Aufgabe löst nach einem ersten Aspekt der Erfindung ein Implantat gemäß Anspruch 1.

Das beanspruchte Implantat ist in Gestalt eines Stützkörpers für die Geweberegeneration, insbesondere die Knochen- oder Weichgewebsregeneration, wobei der Stützkörper ein Versteifungselement und eine Membran aufweist, wobei das Versteifungselement plastisch verformbar ist und Magnesium und/oder eine Magnesiumlegierung aufweist, und wobei die Membran eine flächige Struktur bereitstellt, zum (i) Schutz gegen das Durchwachsen von Gewebe oder zum gezielten Passierenlassen von bestimmten Stoffen oder Substanzen und/oder (ii) Fördern des Anwachsens bestimmer Gewebearten bei bioaktiver Gestaltung oder in bioinerter Gestaltung.

Ein oder mehrere Versteifungselemente können auch als Stütz- oder besser als Versteifungsstruktur bezeichnet werden.

Unter einer Membran ist in Vlies zu verstehen gemäß der beanspruchten Erfindung. Vliese lassen sich hervorragend als Membranen mit gezielt steuerbaren Eigenschaften herstellen.

Beispielsweise kann eine bioinerte Membran, d.h. Vlies, verwendet werden. Eine bioinerte Membran wird dabei als physikalische Barriere idealerweise so appliziert, dass die Membran einen Hohlraum zum Knochen bildet und im Randbereich des Knochendefektes einen direkten Kontakt zur Knochenoberfläche aufweist. Dadurch werden schnell wachsende Zellen des bedeckenden Weichgewebes an der Proliferation in den Defekt hinein gehindert, und nur die sich langsam vermehrenden gefäß- und knochenbildenden Zellen erhalten Zugang.

Die Formulierung "ein Versteifungselement" soll in der Folge stets als "ein oder mehrere Versteifungselemente" verstanden werden, da das Vorhandensein mehrerer Versteifungselemente stets auch das Vorhandensein eines einzelnen Versteifungselements voraussetzt. Generell sei betont, dass Angaben mit unbestimmten Artikeln wie "ein ...", "zwei ..." usw. als mindestens-Angaben zu verstehen sein sollen, also als "mindestens ein ...", "mindestens zwei ..." usw., soweit nicht etwa im konkreten Fall "genau ein...", "genau zwei ..." usw. aus dem Kontext als die Lehre der Aussage hervorgeht.

Der vorgestellte Aspekt der Erfindung ermöglicht eine Verbesserung gegenüber den bisher bekannten Stützkörpern sowohl aus nicht resorbierbaren Metallen, wie beispielsweise Titan, als auch aus resorbierbaren Kunststoffen. Während sich Titan zwar plastisch verformen lässt, aber nicht resorbiert wird, das heißt später wieder entfernt werden muss, können resorbierbare Kunststoffe nicht plastisch verformt werden, das heißt, sie müssen bereits die Form für die jeweilige, jedoch immer individuelle, Applikation am Knochendefekt aufweisen. Ein Stützkörper mit einem Versteifungselement aus Magnesium oder einer Magnesiumlegierung hingegen resorbiert und ist zusätzlich plastisch verformbar.

In Frage kommen sowohl Magnesiumlegierungen, die für eine gegenüber Reinmagnesium verminderte Degradationsrate bekannt sind, wie z.B. WE43, als auch Rein- bzw. Reinstmagnesium oder auch andere Magnesiumlegierungen.

Idealerweise besteht das Versteifungselement vollständig aus Magnesium oder der Magnesiumlegierung.

Auch eine Gestaltung, in welcher das Versteifungselement das Magnesium oder die Magnesiumlegierung nur aufweist, nicht aber vollständig daraus besteht, kann aber bei geeigneter Gestaltung gut geeignet sein. Dies gilt insbesondere dann, wenn das Versteifungselement einen Teil aufweist, welcher als solcher vollständig aus Magnesium oder einer Magnesiumlegierung besteht.

Unter einem "Stützkörper" ist jede dreidimensionale Struktur zu verstehen, die im Rahmen der Geweberegeneration, insbesondere der Knochen- und Weichgewebsregeneration, eingesetzt werden kann und soll, um einen Spalt oder einen Hohlraum auszufüllen, dabei mit der Membran zumindest teilweise abzudecken und die Form von benachbartem Gewebe aufrecht zu erhalten. Der Stützkörper kann mengen- und/oder volumenmäßig betrachtet überwiegend auch aus dem Versteifungselement alleine bestehen.

Unter einem Versteifungselement ist jedes in oder an einem Stützkörper angeordnete Element zu verstehen, das dessen Stabilität erhöht.

Bevorzugt ist das Versteifungselement auf manuelle Weise plastisch verformbar, vor allem mit im chirurgischen Eingriff praktikal aufbringbaren Kräften, so beispielsweise mit Kräften zwischen 1 N, oder besser 3 N, und 50 N, mitunter können auch Kräfte bis 100 N Sinn machen. Der Operateur kann dann während des Eingriffs das Implantat, genauer dessen Versteifungselement, plastisch so verformen, dass es bestmöglich für die zu schützende Stelle passt.

Das Implantat kann mit einer Oberflächenbeschichtung versehen sein, um variabler an den jeweiligen Einsatzort und -zweck anpassbar zu sein.

Das Versteifungselement kann beispielsweise draht-, faden-, band- oder stabförmig ausgeführt sein, sofern es nur eine ausreichende Stabilität aufweist.

Von Vorteil ist, wenn das Versteifungselement draht- oder stabförmig ist.

Besonders vorteilhaft ist es, wenn ein oder mehrere Versteifungselemente als Gitter, Gewebe, Gelege oder Gewirk angeordnet sind und/oder miteinander verbunden sind. Dies gilt sowohl für eine Anordnung einzelner Versteifungselemente, bei denen wie bei einem Textil die einzelnen Elemente an den Berührungspunkten keinen stoffschlüssigen Verbund aufweisen, als auch Anordnungen, bei denen ein solcher stoffschlüssiger Verbund gegeben ist, als auch einzelne Versteifungselemente, das heißt Gitter, die aus einem einzigen Magnesiumkörper bestehen und Aussparungen (Löcher) aufweisen. Ein solcher einzelner Magnesiumkörper kann in seiner Ausprägung als Gitter beliebig viele zweidimensionale Aussparungen verschiedener Form, zum Beispiel rund oder elliptisch oder als beliebiges Vieleck ausgeprägt, und in beliebiger Anordnung zueinander aufweisen. Unter Umständen sieht ein solches Gitter textilen Anordnungen (zum Beispiel einem Gewebe) in seiner Gestalt ähnlich. Ein solcher einzelner Magnesiumkörper kann in seiner Ausprägung als dreidimensionaler Körper beliebig viele Löcher, zum Beispiel durchgehend als "Kanäle" mit beliebiger Querschnittsfläche zum Beispiel rund oder elliptisch oder als beliebiges Vieleck ausgeprägt, in beliebiger Anordnung zueinander und in alle drei Achsrichtungen aufweisen. In Form von Drahtelementen oder kleinen Stäben aus Magnesium können die Versteifungselemente beispielsweise in jeder Kombination, das heißt sowohl als einzelne Elemente als auch kombiniert als Gitter, Masche, Gewebe, Gelege, Mesh, Scaffold, oder ähnliches verwendet werden. Dieses Versteifungselement beziehungsweise diese Versteifungselemente können eine dreidimensionale Form aufweisen, oder in eine solche verbiegbar sein. Die Versteifungselemente aus Magnesium werden resorbiert und ein zweiter Eingriff zur Entnahme der Versteifungselemente entfällt.

Insbesondere kann das Versteifungselement als dreidimensionaler Körper ausgeführt sein. Unter einem dreidimensionalen Körper ist insbesondere eine gitterartige Skelettstruktur zu verstehen.

Auch ein zweidimensionaler Körper ist möglich.

Von Vorteil ist, wenn das Versteifungselement eine oder mehrere Schichten aufweist. Insbesondere eine Schicht, die durch Anodisation oder plasmachemische Anodisation unter Funkenbildung erzeugt ist, oder ein resorbierbares Polymer. So kann die Resorption gesteuert bzw. hinausgezögert werden, indem das Magnesium optional mit einer Beschichtung versehen wird.

Insbesondere durch Anodisation oder plasmachemische Anodisation unter Funkenbildung wird eine Schicht mit keramischen Eigenschaften auf der Oberfläche erzeugt, die chemisch stabiler ist und so den Abbau gezielt beeinflusst. Alternativ können auch andere oder kombinierte Schichtsysteme benutzt werden, z.B. organische Polymerbeschichtungen.

Vorteilhafterweise kann die Oberfläche eine oder mehrere Schichten, insbesondere eine Kalziumphosphatschicht und/oder eine Oxidschicht, aufweisen. Bei der Kalziumphosphatschicht kann es sich zum Beispiel um Hydroxylapatit oder α- oder β-Trikalziumphosphat handeln. Bei der Oxidschicht kann es sich beispielsweise um Magnesiumoxid oder andere Mischoxide handeln. Diese Stoffe entstehen typischerweise im Rahmen der Anodisation oder der plasmachemischen Anodisation oder werden im Rahmen dieser als Partikel in die Schicht fest mit eingebaut, wobei das Magnesium oxidiert wird. Die Oxidschicht ist fest mit dem Grundkörper verbunden. Durch die chemische Stabilität der Schicht verbessert die Schicht den Schutz vor korrosivem Angriff an der Oberfläche und beeinflusst gezielt die Resorption. In einer Schicht können die oben genannten Stoffe auch gemischt vorliegen. Beliebige Schichten können auch hintereinander in beliebiger Kombination auftreten.

Die zuletzt aufgetragene also äußerste Schicht kann auch aus einer organischen Beschichtung, insbesondere einem organischen Polymer und/oder aus einer anorganischen Beschichtung, insbesondere einem anorganischen Polymer bestehen beziehungsweise kann derartige Bestandteile enthalten.

Beispielsweise sei an ein Sol-Gel gedacht: Es kann ein Top-Coating in Form einer Sol-Gel-Beschichtung vorgenommen werden, insbesondere mit einem ein Silan oder einem Epoxy-Silan. Nach dem Brennen im Ofen verbleibt nur der anorganische Teil, also beispielsweise das Silan, welches glas- oder jedenfalls siliziumähnliche Eigenschaften haben kann; wenn nicht so heiß gebrannt wird, dann verbleibt Epoxy-Silan.

Des Weiteren kann diese Schicht mit organischen oder anorganischen Wirksubstanzen beladen sein, so zum Beispiel Zelladhäsionsmoleküle zur Optimierung der Zellnische, Zytokine (Wachstums- oder Differenzierungsfaktoren) und virale beziehungsweise non virale Genvektoren (u.a. Plasmide).

Hierin wird auch ein Implantat mit einem Versteifungselement in Gestalt eines Fadens, Bands oder Drahts, wobei der Stützkörper manuell plastisch verformbar ist, und wobei das Implantat Magnesium oder eine Magnesiumlegierung als Werkstoff für das Versteifungselement und zusätzlich eine antibakterielle Beschichtung aufweist beschrieben.

Die Beschichtung kann bevorzugt direkt auf dem Magnesium bzw. der Legierung oder auf einer anodisierten Oberfläche aufgetragen sein.

Ein dreidimensionaler Körper kann vor allem so konstruiert sein, dass ein oder mehrere Drähte, Fäden, Bänder und/oder Stäbe als Gitter, Gewebe, Gelege oder Gewirk oder beispielsweise Getrick oder Geflecht angeordnet sind und/oder miteinander verbunden sind.

Von Vorteil ist es, wenn der Stützkörper eine Trägersubstanz, beispielsweise für eine antibakterielle Beschichtung, aufweist, insbesondere ein natives oder künstliches Kollagen oder ein Fibrin oder eine natürliche oder künstliche Seide oder ein anderes natürliches Protein oder ein Polymer, wie beispielsweise PLA oder PTFE.

Vorteilhafter Weise kann eine Membran aus einer Trägersubstanz Taschen aufweisen, die in die Membran eingearbeitet sind. So kann eine Verbindung einer resorbierbaren Membran mit einer Struktur aus Versteifungselementen durch Einstecken dieser in die Taschen erfolgen, wie es bei den Verstrebungen eines Drachens, die in die Ecken eines Drachens eingesteckt werden, erfolgt. Alternativ kann die Stützstruktur aufgeklebt bzw. in den Stützkörper eingeklebt werden, durch Niet-, Klammer, oder Steckverbindungen befestigt, mit einem unter Umständen, resorbierbaren Faden aufgenäht oder während des Herstellungsprozesses in den Stützkörper wie zum Beispiel eine Membran eingearbeitet werden. Letztes bedeutet, dass das Material des Stützkörpers um die Stützstruktur herum, aufgebaut wird.

Weiter von Vorteil ist es, wenn eine äußerste Schicht eine antibakterielle Substanz oder ein Antibiotikum aufweist. Auch eine Membran kann mit einer biofunktionalen Substanz, beispielsweise antibakteriell beladen sein.

Nach einem weiteren Aspekt der hier vorliegenden Erfindung hat das Implantat die Gestalt eines Myokardpatches, wobei das Implantat einen Stützkörper in Form eines Versteifungselements aufweist, wobei das Versteifungselement manuell plastisch verformbar ist und Magnesium und/oder eine Magnesiumlegierung aufweist.

Es sei ausdrücklich darauf hingewiesen, dass ein Implantat in Gestalt eines Myokardpatches weitere Eigenschaften und Merkmale der übrigen im Rahmen der hier vorliegenden Patentanmeldung beschriebenen Implantate ebenfalls aufweisen kann.

So können unterschiedliche Anforderungen im Rahmen der Geweberegeneration insbesondere der Knochen- und Weichgewebsregeneration erfüllt werden. So kann beispielsweise durch Myokardpatches ein durch einen Herzinfarkt abgestorbenes Gewebe an der Herzwand durch einen Stützkörper, z.B. künstliches, mit Magnesium verstärktes Gewebe stabilisiert werden.

Ein weiterer Aspekt der Erfindung betrifft ein Verfahren zur Herstellung eines Implantats wie vorstehend beschrieben. Dabei kann ein Versteifungselement innen oder außen eingenäht, eingewebt, eingesteckt, eingeklebt, eingelegt oder anderweitig in die Membran eingearbeitet werden.

Bei einem Verfahren zur Herstellung einer Abdeckmembran kann vorteilhafterweise ein Versteifungselement als mittlere Schicht eingearbeitet, insbesondere eingepresst werden.

In den Figuren 1, 2 und 3 sind Referenzausführungen, nicht gemäß der beanspruchten Erfindung, dargestellt. Figur 4 zeigt Strukturen gemäß der beanspruchten Erfindung. Hierin zeigen
- Figur 1: in schematischer räumlicher Ansicht einen resorbierbaren Faden,
- Figur 2: schematisch die Verwendung eines resorbierbaren Fadens im Zahnsulcus
- Figur 3: beispielhafte mögliche Designs für Versteifungselemente und
- Figur 4: eine Kollagenmembran mit resorbierbarer Stützstruktur aus Magnesium.

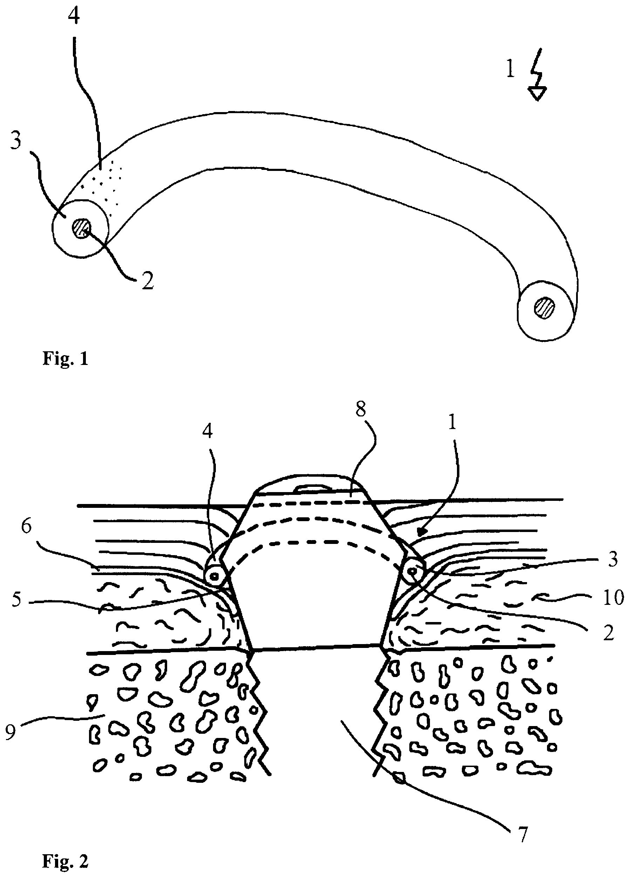

Der resorbierbare Faden 1 in Figur 1 weist eine Magnesiumseele 2 auf, die von einer Trägersubstanz 3 umgeben ist. Auf der Oberfläche der Trägersubstanz 3 befindet sich eine biofunktionale Beladung, beispielsweise in Form einer antibakteriellen oder antibiotischen Substanz 4. Dieser Faden 1 wird wie in Figur 2 dargestellt in den Sulcus 5 zwischen Gingiva 6 und Implantat 7 mit Abutment 8 appliziert. Hierbei wird das Implantat 7 von Knochen 9 und superkrestalem Bindegewebe 10 umschlossen. Durch die kontrollierte Freisetzung der antibakteriellen Substanz direkt im Sulcus kann so eine gezielte Therapie erfolgen.

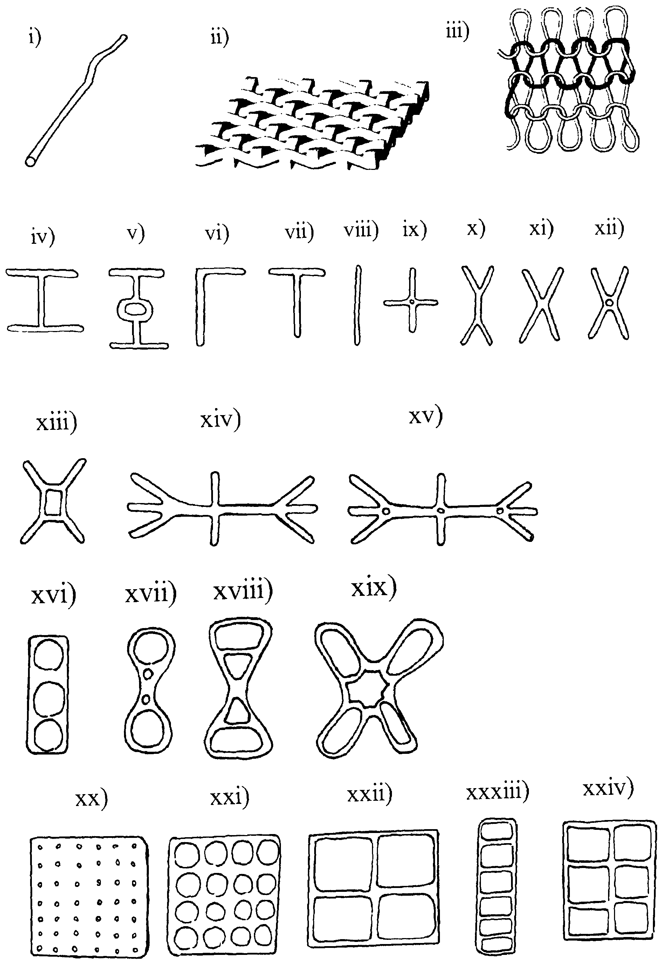

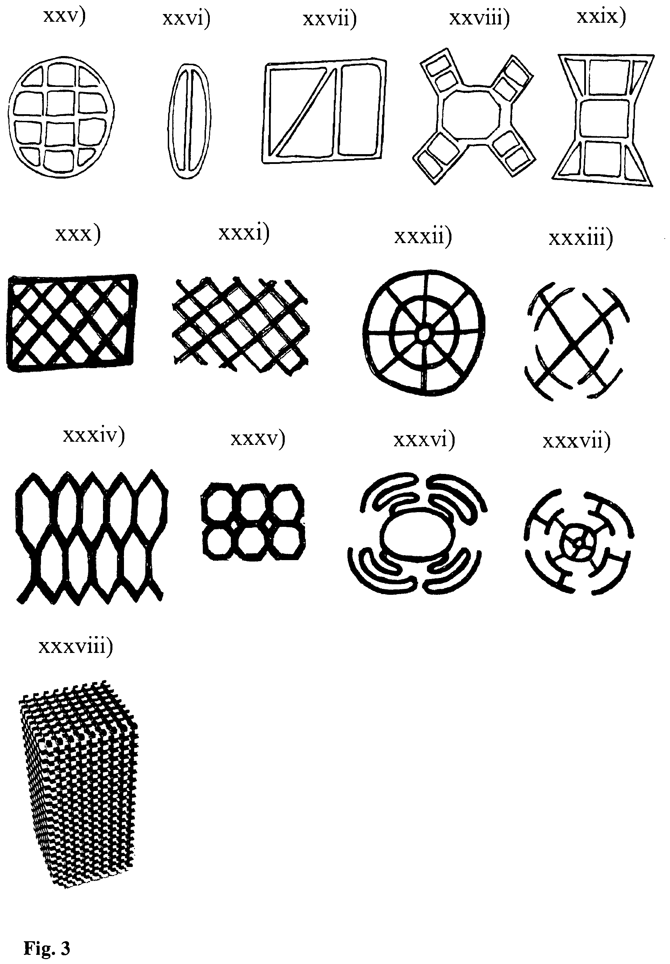

Versteifungselemente können wie in Figur 3 abgebildet zahlreiche Formen aufweisen. So können eher stabförmige Strukturen ausgebildet werden, die unter Umständen zu mehreren als Netz- oder Gewebestrukturen wie ein Textil mit einander verbunden werden. Es können auch eher plattenartige Strukturen als zusammenhängende Gitter, das heißt als einzelne Körper mit Aussparungen, ausgebildet werden. Die Formenkönnen symmetrisch oder asymmetrisch sein. Insbesondere kann es sich um einen Draht (i) ein Gewebe (ii) oder ein Gewirk (iii) um Stabformen (iv) bis (ix), Gitterformen (xx bis xxxvii) oder um eine dreidimensionale Skelettstruktur (xxxviii) handeln.

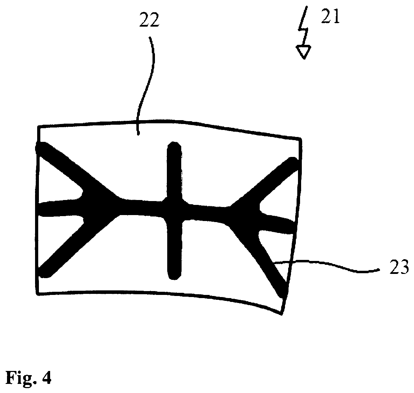

Bei dem Stützkörper (21) in Figur 4 wird eine Kollagenmembran (22) durch das Versteifungselement (23) gestützt. Dieses Versteifungselement (23) ist biegbar, sodass dem Versteifungselement (23) und damit der Zusammensetzung aus Kollagenmembran (22) und Versteifungselement (23) als Stützkörper (21) eine quasi beliebige Form gegeben werden kann.

## Patentansprüche

1. Implantat in Gestalt eines Stützkörpers für die Geweberegeneration, insbesondere die Knochen- oder Weichgewebsregeneration,
wobei das Implantat dazu einsetzbar ist, im Rahmen der Geweberegeneration eingesetzt zu werden und um einen Spalt oder einen Hohlraum auszufüllen, dabei mit einer Membran zumindest teilweise abzudecken und die Form von benachbartem Gewebe aufrecht zu erhalten,
wobei das Implantat die Membran und ein Versteifungselement zum Stützen der Membran aufweist, so dass die Membran mittels des Versteifungselements gestützt ist,
wobei das Versteifungselement Magnesium und/oder eine Magnesiumlegierung aufweist, so dass das Versteifungselement plastisch verformbar ist,
wobei die Membran eine flächige Struktur bereitstellt, zum (i) Schutz gegen das Durchwachsen von Gewebe oder zum gezielten Passierenlassen von bestimmten Stoffen oder Substanzen und/oder (ii) Fördern des Anwachsens bestimmter Gewebearten bei bioaktiver Gestaltung oder in bioinerter Gestaltung, und
wobei die Membran resorbierbar und ein Vlies ist, so dass das Implantat in Form eines Stützkörpers resorbiert und plastisch verformbar ist.

2. Implantat nach Anspruch 1, ***dadurch gekennzeichnet, dass*** das Implantat die Gestalt eines Myokardpatches hat, wobei das Versteifungselement manuell plastisch verformbar ist.

3. Implantat nach Anspruch 1 oder 2, ***dadurch gekennzeichnet, dass*** das Implantat eine Oberflächenbeschichtung aufweist.

4. Implantat nach einem der vorstehenden Ansprüche, ***dadurch gekennzeichnet, dass*** das Versteifungselement draht-, faden-, band- oder stabförmig ausgeführt ist.

5. Implantat nach Anspruch 4, ***dadurch gekennzeichnet, dass*** mehrere Versteifungselemente als Gitter, Gewebe, Gelege oder Gewirk angeordnet sind und/oder miteinander verbunden sind.

6. Implantat nach einem der vorstehenden Ansprüche, ***dadurch gekennzeichnet, dass*** das Versteifungselement als dreidimensionaler Körper ausgeführt ist.

7. Implantat nach einem der vorstehenden Ansprüche, ***dadurch gekennzeichnet, dass*** das Versteifungselement eine oder mehrere Schichten aufweist.

8. Implantat nach Anspruch 7, ***dadurch gekennzeichnet, dass*** das Versteifungselement eine Kalziumphosphatschicht und/oder eine Oxidschicht oder eine Schicht, die durch Anodisation oder plasmachemische Anodisation unter Funkenbildung erzeugt ist aufweist.

9. Implantat nach Anspruch 1 oder einem der Ansprüche 3 bis 8, ***dadurch gekennzeichnet, dass*** eine äußerste Schicht des Implantats aus einer organischen Beschichtung besteht, insbesondere aus einem organischen Polymer und/oder aus einer anorganischen Beschichtung, insbesondere einem anorganischen Polymer.

10. Implantat nach Anspruch 4, ***dadurch gekennzeichnet, dass*** ein oder mehrere Drähte, Fäden, Bänder und/oder Stäbe als Gitter, Gewebe, Gelege oder Gewirk angeordnet sind und/oder miteinander verbunden sind.

11. Implantat nach Anspruch 1 oder einem der Ansprüche 3 bis 9, ***dadurch gekennzeichnet, dass*** der Stützkörper eine Trägersubstanz für eine antibakterielle Beschichtung aufweist, insbesondere ein natives oder künstliches Kollagen oder ein Fibrin oder eine natürliche oder künstliche Seide oder ein anderes natürliches Protein oder ein Polymer, wie beispielsweise PLA oder PTFE.

12. Verfahren zum Herstellen eines Implantats nach Anspruch 1 oder einem der Ansprüche 3 bis 9, ***dadurch gekennzeichnet, dass*** beim Formen des Stützkörpers das Versteifungselement als eine mittlere Schicht in die Membran eingepresst oder anderweitig eingearbeitet wird.

13. Verfahren zum Herstellen eines Implantats nach Anspruch 1 oder einem der Ansprüche 3 bis 9, ***dadurch gekennzeichnet, dass*** das Versteifungselement innen oder außen eingenäht, eingewebt, eingesteckt, eingeklebt, eingelegt oder anderweitig in die Membran eingearbeitet wird.

## Claims

1. Implant in the form of a support body for tissue regeneration, in particular bone or soft tissue regeneration, wherein the implant can be used in the context of tissue regeneration and in order to fill a gap or cavity, at least partially covering said gap or cavity with a membrane and maintaining the shape of adjacent tissue, wherein the implant comprises the membrane and a reinforcing element for supporting the membrane such that the membrane is supported by means of the reinforcing element, wherein the reinforcing element comprises magnesium and/or a magnesium alloy such that the reinforcing element is plastically deformable, wherein the membrane provides a flat structure, for (i) protection against the intermixing of tissue or for the targeted passage of particular matter or substances and/or (ii) promoting the growth of particular types of tissue in bioactive design or in bioinert design, and wherein the membrane is resorbable and a nonwoven fabric such that the implant in the form of a support body is resorbed and plastically deformable.

2. Implant according to claim 1, ***characterized in that*** the implant is in the form of a myocardial patch, the reinforcing element being manually plastically deformable.

3. Implant according to either claim 1 or claim 2, ***characterized in that*** the implant comprises a surface coating.

4. Implant according to any of the preceding claims, ***characterized in that*** the reinforcing element is in the shape of a wire, thread, strip or rod.

5. Implant according to claim 4, ***characterized in that*** several reinforcing elements are provided in the form of a mesh, woven fabric, laid scrim or knitted fabric and/or are interconnected.

6. Implant according to any of the preceding claims, ***characterized in that*** the reinforcing element is designed as a three-dimensional body.

7. Implant according to any of the preceding claims, ***characterized in that*** the reinforcing element comprises one or more layers.

8. Implant according to claim 7, ***characterized in that*** the reinforcing element comprises a calcium phosphate layer and/or an oxide layer, or a layer which is created by anodization or plasma-chemical anodization with sparking.

9. Implant according to claim 1 or any of claims 3 to 8, ***characterized in that*** an outermost layer of the implant consists of an organic coating, in particular an organic polymer, and/or of an inorganic coating, in particular an inorganic polymer.

10. Implant according to claim 4, ***characterized in that*** one or more wires, threads, strips and/or rods are provided in the form of a mesh, woven fabric, laid scrim or knitted fabric and/or are interconnected.

11. Implant according to claim 1 or any of claims 3 to 9, ***characterized in that*** the support body comprises a carrier substance for an antibacterial coating, in particular a native or artificial collagen or a fibrin or a natural or artificial silk or another natural protein or a polymer, such as PLA or PTFE.

12. Method for producing an implant according to claim 1 or any of claims 3 to 9, ***characterized in that*** when the support body is formed, the reinforcing element is pressed, or is otherwise incorporated, into the membrane as a middle layer.

13. Method for producing an implant according to claim 1 or any of claims 3 to 9, ***characterized in that*** the reinforcing element is sewn, woven, inserted, glued, laid or otherwise incorporated into the membrane on the inside or outside.

## Revendications

1. Implant se présentant sous la forme d'un corps de support pour la régénération tissulaire, en particulier la régénération osseuse ou la régénération des tissus mous,
où l'implant, dans le cadre de la régénération tissulaire, peut être utilisé et mis en place pour combler une fissure ou une cavité et, ce faisant, pour recouvrir au moins partiellement avec une membrane et pour conserver la forme de tissus adjacents,
où l'implant présente la membrane et un élément de renfort servant à supporter la membrane, de sorte que la membrane est supportée au moyen de l'élément de renfort,
où l'élément de renfort contient du magnésium et/ou un alliage de magnésium, de sorte que l'élément de renfort est plastiquement déformable,
où la membrane fournit une structure plate, servant (i) à la protection contre la croissance pénétrante de tissu ou pour laisser passer, de manière ciblée, certaines matières ou substances et/ou servant (ii) à favoriser la croissance de certains types de tissus dans le cas d'une configuration bioactive ou dans une configuration bio-inerte, et
où la membrane est résorbable et un non-tissé, de sorte que l'implant est résorbé sous la forme d'un corps de support et est plastiquement déformable.

2. Implant selon la revendication 1, ***caractérisé en ce que*** l'implant a la forme d'un patch myocardique, où l'élément de renfort est plastiquement déformable manuellement.

3. Implant selon la revendication 1 ou 2, ***caractérisé en ce que*** l'implant présente un revêtement de surface.

4. Implant selon l'une quelconque des revendications précédentes, ***caractérisé en ce que*** l'élément de renfort est conçu sous la forme d'un fil métallique, d'un fil textile, d'une bande ou d'une tige.

5. Implant selon la revendication 4, ***caractérisé en ce que*** plusieurs éléments de renfort sont disposés comme une grille, un tissu, un canevas ou un tricot et/ou sont reliés les uns aux autres.

6. Implant selon l'une quelconque des revendications précédentes, ***caractérisé en ce que*** l'élément de renfort est conçu comme un corps tridimensionnel.

7. Implant selon l'une quelconque des revendications précédentes, ***caractérisé en ce que*** l'élément de renfort présente une ou plusieurs couches.

8. Implant selon la revendication 7, ***caractérisé en ce que*** l'élément de renfort présente une couche de phosphate de calcium et/ou une couche d'oxyde, ou bien une couche qui est produite par anodisation ou par anodisation plasmachimique avec formation d'étincelles.

9. Implant selon la revendication 1 ou l'une quelconque des revendications 3 à 8, ***caractérisé en ce qu'une*** couche de l'implant, placée le plus à l'extérieur, se compose d'un revêtement organique, en particulier d'un polymère organique et/ou d'un revêtement minéral, en particulier d'un polymère minéral.

10. Implant selon la revendication 4, ***caractérisé en ce* qu'**un ou plusieurs fils métalliques, fils textiles, une ou plusieurs bandes et/ou tiges, sont disposés comme une grille, un tissu, un canevas ou un tricot et/ou sont reliés les uns aux autres.

11. Implant selon la revendication 1 ou l'une quelconque des revendications 3 à 9, ***caractérisé en ce que*** le corps de support contient une substance porteuse pour un revêtement antibactérien, en particulier un collagène natif ou artificiel ou une fibrine ou une soie naturelle ou artificielle ou une autre protéine naturelle, ou bien un polymère comme par exemple du PLA ou du PTFE.

12. Procédé de fabrication d'un implant selon la revendication 1 ou l'une quelconque des revendications 3 à 9, ***caractérisé en ce que*,** lors du formage du corps de support, l'élément de renfort est pressé dans la membrane comme une couche intermédiaire ou bien incorporé autrement.

13. Procédé de fabrication d'un implant selon la revendication 1 ou l'une quelconque des revendications 3 à 9, ***caractérisé en ce que*** l'élément de renfort est, intérieurement ou extérieurement, cousu, tissé, emboîté, collé, inséré, ou bien incorporé autrement dans la membrane.
